# EUROPÄISCHE PATENTSCHRIFT

(11) **EP 1 218 242 B1**
(45) Veröffentlichungstag und Bekanntmachung des Hinweises auf die Patenterteilung: **01.12.2004**
(21) Anmeldenummer: 00969427.4
(22) Anmeldetag: 05.10.2000
(51) Int. Cl.: B64D 25/00, B64D 10/00, A61F 5/055, A61F 5/01

(54) **EINRICHTUNG ZUR ENTLASTUNG DER HALSWIRBELSÄULE**
DEVICE FOR SUPPORTING THE VERTEBRAL COLUMN
DISPOSITIF DESTINE A SOULAGER LA COLONNE CERVICALE

(30) Priorität: 05.10.1999 DE 19947841; 09.12.1999 DE 19959313
(43) Veröffentlichungstag der Anmeldung: 03.07.2002
(73) Patentinhaber: Schmitz, Josef, 83083 Riedering (DE)
(72) Erfinder: Schmitz, Josef, 83083 Riedering (DE)
(74) Vertreter: von Puttkamer, Nikolaus, Dipl.-Ing.
(86) Internationale Anmeldenummer: PCT/EP2000/009759
(87) Internationale Veröffentlichungsnummer: WO 2001/025088

(56) Entgegenhaltungen:
- WO-A-99/03440
- DE-A- 3 318 938
- DE-A- 3 905 115
- DE-A- 19 849 302
- DE-U- 1 914 724
- US-A- 5 039 035
- US-A- 5 752 927

## Beschreibung

Die vorliegende Erfindung betrifft eine Einrichtung zur Entlastung der Halswirbelsäule nach dem Oberbegriff des Patentanspruches 1.

Insbesondere im Zusammenhang mit dem Ausschuß von Schleudersitzen aus Düsenflugzeugen sind verschiedene Einrichtungen zur Entlastung des Körpers eines Besatzungsmitgliedes bekannt geworden. Beispielsweise ist in der DE 40 02 215 C1 eine Einrichtung zur Entlastung des Körpers eines Besatzungsmitgliedes eines Luft- oder Bodenfahrzeuges beschrieben, die einen Entlastungsseilzug umfaßt, der einerseits mit einer Anlenkung am Schutzhelm bzw. an einem am Schutzhelm angeordneten Ausrüstungsgegenstand angreift und andererseits an ein elastisch nachgiebiges Element anschließt, das sich an feststehenden Teilen des Luftfahrzeuges abstützt und ständig auf den Körper des Besatzungsmitgliedes mit einer Rückhaltekraft einwirkt. Ein Problem einer solchen Entlastungseinrichtung besteht darin, dass einerseits eine andauernde Verbindung zwischen dem Helm und dem Luftfahrzeug besteht, sodass die Bewegungsfreiheit des Besatzungsmitgliedes stark eingeschränkt ist und dass andererseits eine wirksame Entlastung der Halswirbelsäule des Besatzungsmitgliedes nicht erfolgen kann, weil der genannte Entlastungsseilzug keine den auftretenden G-Kräften entgegenwirkende Kraft übertragen kann.

Bei anderen bekannten Entlastungsvorrichtungen wird das Besatzungsglied jeweils fest mit dem Schleudersitz verbunden, was ebenfalls problematisch ist, weil aufgrund dieser festen Verbindung Vibrationskräfte auf den Körper, insbesondere über den Kopf auf die Halswirbelsäule übertragen werden.

Aus der US-A-5,752,927 geht eine orthopädische Einrichtung zum Stützen der Halswirbelsäule mit den Merkmalen des Oberbegriffes des Patentanspruches 1 hervor, wobei vier getrennte Luftkammern, nämlich zwei vordere Luftkammern und zwei hintere Luftkammern, vorgesehen sind, die sich mit ihren einen Seiten an der Schulter eines Benutzers abstützen. Die beiden vorderen Luftkammern stützen sich mit ihren anderen Seiten am Hals des Benutzers ab. Die beiden hinteren Luftkammern stützen sich mit ihren anderen Seiten an der Hinterhauptschuppe des Benutzers ab. Die vier Luftkammern sind mit der Hilfe von hand- oder fußbetätigten Luftpumpen aufpumpbar.

Die Aufgabe der vorliegenden Erfindung besteht darin, eine aktive Einrichtung zur Entlastung der Halswirbelsäule eines Menschen beim Fahren in Fahrzeugen zu schaffen, die einen Schutz der Halswirbelsäule bewirken kann.

Diese Aufgabe wird durch eine Einrichtung mit den Merkmalen des Patentanspruches 1 gelöst.

Der wesentliche Vorteil besteht darin, dass die erfindungsgemäße Einrichtung zur Entlastung der Halswirbelsäule einen umfassenden Schutz sowohl gegen G-Kraft-abhängige Kompressions- und/oder Stauchungs- und Scherungskräfte, wie auch gegen Vibrationen und horizontal und/oder vertikal und/oder paraaxial einwirkende Beschleunigungskräfte liefert, die beispielsweise von einem Fahrzeug, insbesondere einem Düsenflugzeug oder Kampfhubschrauber, auf den Körper des Menschen übertragen werden. Die zuvor angesprochenen Kompressions- und Vibrationsentlastungen spielen insbesondere dann eine ganz wesentliche Rolle, wenn der vor diesen Kompressionen und Vibrationen zu schützende Mensch einen Helm trägt, der insbesondere auf dem militärischen Sektor noch mit zusätzlichen Ausrüstungsgegenständen, wie beispielsweise Visier oder Nachtsichtgerät, versehen ist und daher ein großes Gewicht aufweist. Durch den Einsatz der erfindungsgemäßen Einrichtung können Schleudertraumen, wie lageabhängige Distorsionen, ligamentäre Überbeanspruchungen und Blockierungen, Bandscheibenüberbelastungen, Verletzungen der arteriellen Gefäße (Arteria-vertebralis-Dissektion) und daraus resultierende degenerative Veränderungen des Achsenskelettes weitgehend vermieden werden.

Vorteilhafterweise ist die erfindungsgemäße Einrichtung so gestaltet, dass sie die völlige Beweglichkeit des Kopfes und des Unterkiefers auch beim Einwirken von G-Kräften erhält. Eine Verbindung zwischen dem Kopf bzw. Helm und dem Fahrzeug besteht nicht.

Da große Helmgewichte beim Einsatz der erfindungsgemäßen Einrichtung kompensiert werden können, wird die Flexibilität bei der Herstellung und Entwicklung von Helmen und/oder von an Helmen anzubringenden Zusatzgeräten wesentlich erhöht. Insbesondere können durch Zusatzgeräte bewirkte Helmgewichte auch bei einer ungünstigen Schwerpunktlage kompensiert werden.

Ein weiterer wesentlicher Vorteil besteht darin, dass es die vorliegende Einrichtung ermöglicht, dass insbesondere bei Schleudersitzausschüssen der Kopf des durch die Einrichtung geschützten Menschen automatisch in eine optimale Haltung gebracht wird, sodass ungünstige Belastungsspitzen im Bereich der Halswirbelsäule verhindert werden.

Die vorliegende Einrichtung bietet vorteilhafterweise einen Schutz gegen exogene Verletzungen, wie sie beispielsweise bei Ausschüssen mit einem Schleudersitz aus einem Jet durch Fremdkörper und Splitter sowie bei Vogelschlag während des Fluges oder unter Beschuß mit Feuerwaffen auftreten können.

Da die hohen Helmgewichte sowie die ungünstigen Helm-Schwernunktlagen durch die erfindungsgemäße Einrichtung kompensiert werden, können subjektive Beschwerden, wie Kopfschmerzen und andere Teilleistungsstörungen verhindert werden, die insbesondere bei auftretenden G-Kräften verursacht werden, die eine Vervielfachung des Helmgewichtes bewirken. Auf solche Störungen zurückzuführende Gefahren können deshalb vermieden werden.

Insbesondere bei Hubschrauberpiloten, die mit Nachtsichtgeräten fliegen, kann die vibrationsabsorbierende Einrichtung gemäß der vorliegenden Erfindung vorteilhafter Weise nicht nur zu einer Verminderung der gesundheitlichen Belastungen, sondern auch zu einer Verbesserung der technischen Bedingungen der helmintegrierten Sicht- und Visiersysteme führen.

Von großer Bedeutung ist es, dass die vorliegende Einrichtung individuell an den jeweiligen Benutzer in Abhängigkeit von vorangehenden, beispeilsweise radiologischen Untersuchungen der Halswirbelsäule anpaßbar ist.

Die vorliegende Erfindung eignet sich in einer aktiven, systemgebundenen Version vorzugsweise für Piloten von Düsenflugzeugen, bei Ausschüssen aus Jets, für Kunstflieger, Auto-Rennfahrer und Boot-Rennfahrer.

Weitere Vorteile der Erfindung gehen aus den Unteransprüchen hervor.

Im folgenden werden die Erfindung und deren Ausgestaltungen im Zusammenhang mit den Figuren näher erläutert. Es zeigen:
- Figur 1: in perspektivischer und schematischer Darstellung eine erfindungsgemäße Einrichtung zur Entlastung der Halswirbelsäule;
- Figur 2, 3: eine Vorderansicht bzw. eine Seitenansicht auf die an einen Menschen angelegte Einrichtung der Figur 1 sowie die auf den Kopf des Menschen einwirkenden Kräfte, die durch die Einrichtung erzeugbar sind;
- Figur 4: ein Blockschaltbild zur Erläuterung der Funktion der erfindungsgemäßen Einrichtung;
- Figur 5: eine Weiterbildung der Erfindung;
- Figur 6: eine Darstellung zur Erläuterung des Erfindungsprinzips; und
- Figur 7: eine Weiterbildung der Erfindung.

Zu der Erfindung führten die folgenden Überlegungen. Um die Nachteile des Standes der Technik zu vermeiden und insbesondere einen Schutz der Halswirbelsäule bei nahezu vollständiger Bewegungsfreiheit zu ermöglichen, wurde eine aktive Einrichtung entwickelt, die den Kopf eines Menschen in Bezug auf den Körpei desselben abstützen und lagemäßig justieren kann, ohne dass irgendeine physikalische Verbindung zu, in Bezug auf die Bewegungen des Menschen statischen Teilen, wie beispielsweise Teilen eines Fahrzeuges, bestehen. Die erfindungsgemäße Einrichtung weist dabei im wesentlichen die Form eines am Halsbereich eines Menschen anordenbaren kragenförmigen Elementes auf, das sich auf dem Schulterbereich des Menschen abstützt und verschiedene Druckpolster umfaßt, die mit einem Fluid gefüllt sind und eine von der G-Kraft abhängige Abstützung des Kopfes in Bezug auf die Schulter des Menschen bewirken können.

Durch selektive Veränderung der Drücke in den einzelnen Druckpolstern kann der Kopf des Menschen in Bezug auf die Schulter um eine Querachse verschwenkt werden (Neigen des Kopfes nach vorne oder nach hinten) oder um eine Längsachse verschwenkt werden (Neigen des Kopfes zur Seite nach rechts oder nach links). Zusätzlich ist dafür Sorge getragen, dass der Kopf um die Hochachse nach rechts oder links verdreht werden kann. Auf diese Weise wird eine völlige Bewegungsfreiheit sichergestellt und erreicht, dass die erfindungsgemäße Einrichtung sämtliche Kräfte, die auf den Kopf einwirken, insbesondere Kräfte, die zu Kompressionen der Halswirbelsäule führen können, durch Abstützen des Kopfes auf den Schulterbereichen und auf Vibrationskräfte zurückzuführende Relativbewegungen zwischen Kopf und Körper vermieden werden können.

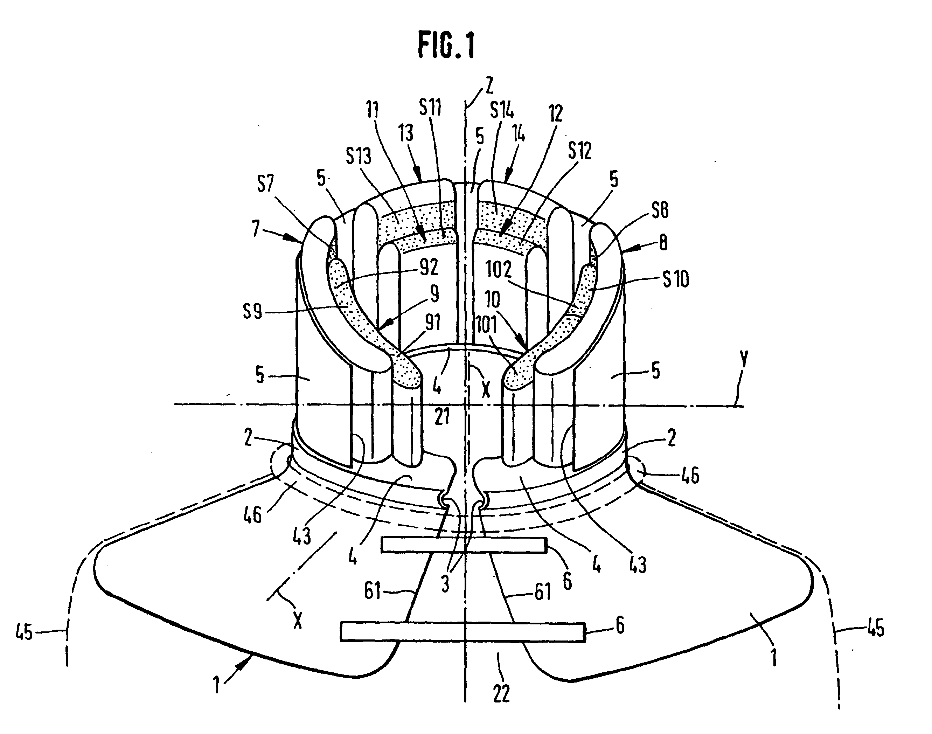

In der aus der Figur 1 ersichtlichen Weise weist die vorliegende Einrichtung zur Entlastung der Halswirbelsäule eine Art Stützschale 1 auf, die vorzugsweise im wesentlichen aus hartem Kunststoff besteht und so geformt ist, dass sie auf die Schulterbereiche eines Benutzers auflegbar ist und sich an diesen möglichst großflächig, vorzugsweise auch auf den Schlüsselbeinbereichen, abstützen kann. An ihrer dem Kopf zugewaudlen Seite weist die Stützschale 1 ein Ringelement 2 auf, das eine Öffnung 21 umschließt, durch die hindurch der Hals des Benutzers verlaufen kann. Um die Anordnung der Stützschale 1 auf der Schulter des Benutzers zu ermöglichen, weist diese beispielsweise einen vorderen Schlitz 22 auf, sodass die Stützschale 1 und das Ringelement 2 so aufweitbar sind, dass sie um den Hals des Benutzers anordenbar sind. Alternativ sind jedoch auch Ausführungsformen denkbar, bei denen die Einrichtung geteilt ist, wobei beispielsweise eine rechte und eine linke Hälfte am Benutzer miteinander verbunden werden. In dem Ringelement 2 ist der untere ebenfalls ringförmige Randbereich 3 einer Stützplatte 4 verdrehbar angeordnet, wobei sich an der Stützplatte 4 ein stehkragenförmiges Wandteil 5 befindet, das sich etwa senkrecht zur Stützplatte 4 erstreckt. Das Wandteil 5 und die Stützplatte 4 sind in Bezug auf die Stützschale 1 um die Achse Z drehbar. Dadurch wird eine Verdrehung des Kopfes ermöglicht.

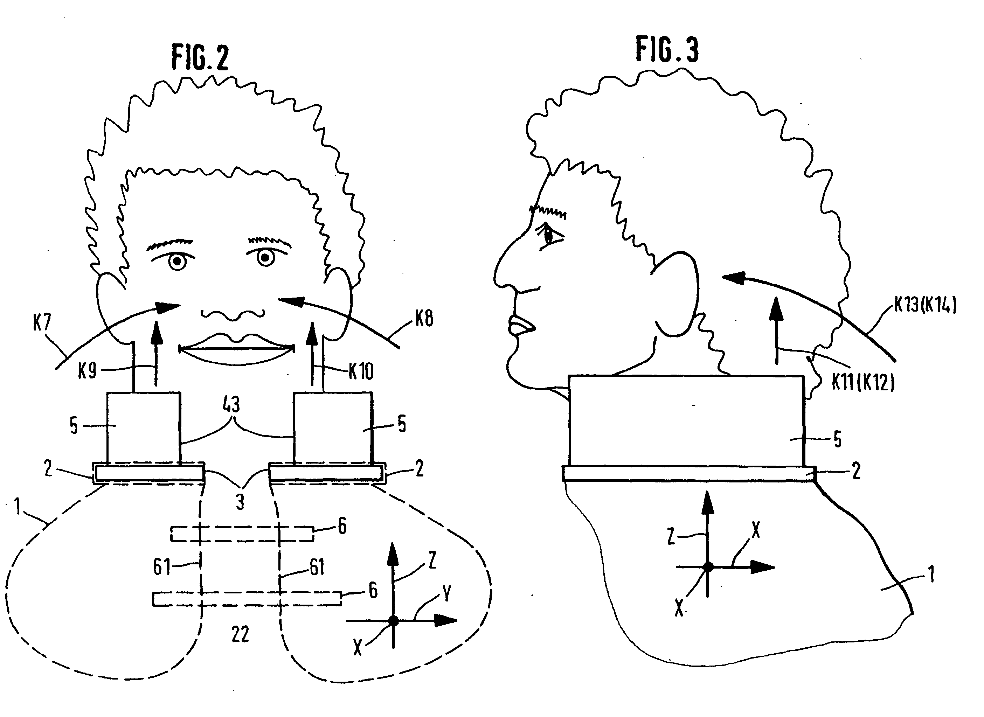

Beispielsweise besitzt gemäß Figur 2 das Ringelement 2 einen U-förmigen Querschnitt, wobei sich die Schenkel des gebildeten "U" zur Öffnung 21 hin erstrecken. In diesen Querschnitt greift von der Seite der Öffnung 21 her der Randbreich 3 ein, der vorzugsweise ebenfalls einen U-förmigen Querschnitt aufweist

Das Wandteil 5 verläuft lediglich über einen Umfangsbereich, der kleiner als 360° ist und eine vordere Aussparung 43 freiläßt, die ein Durchführen des Halses des Benutzers in radialer Richtung ermöglicht.

Der Schlitz 22 der Stützschale 1 ist durch schematisch dargestellte Verschließeinrichtungen 6 verschließbar, die vorzugsweise die Form von Gurten oder Metallklammern aufweisen, bei deren Betätigung in die Schließstellung die Ränder 61 der Stützschale 1 aufeinander zu gezogen werden.

Besonders bevorzugt ist die dichte Verbindung eines Ganzkörperschutzanzuges mit der Hartschale 1. Zu diesem Zweck ist an der Hartschale 1 an der oberen Seite, insbesondere unterhalb des Ringelementes 2 der obere Rand des Ganzkörperschutzanzuges 45 mit einer ringförmigen Dichtungseinrichtung 46 verbunden, die einerseits dicht an der Hartschale 1 anliegt und andererseits dicht mit dem oberen Rand des Ganzkörperschutzanzuges 45 verbunden ist. Die ringförmige Dichtungseinrichtung 46 ist in der Figur 1 lediglich schematisch dargestellt. Sie kann vorzugsweise aus einem Gummimaterial bestehen, das so aufweitbar ist, dass der Kopf eines Piloten beim Aufspreizen der Hartschale 1 durch diese und die Dichtungseinrichtung 46 hindurchführbär ist. Von oben her ist dann der Schutzhelm (nicht dargestellt) dicht mit der oberen Seite des Ganzkörperschutzanzuges 45 verbindbar. Auf diese Weise wird erreicht, dass beim Tragen der vorliegenden Einrichtung, z.B. beim Tragen der vorliegenden Einrichtung, z.B. bei Wasserlandungen nach einem Jetausschuß, kein Wasser in den Anzug eindringen kann, wobei der Überdruck im Anzug erhalten bleibt.

Im Inneren des zweiten Hartschalenteiles 4 sind kissenförmige Druckpolster 7, 8, 9, 10, 11, 12, 13 und 14 angeordnet, die in der später noch näher erläuterten Weise jeweils durch ein Fluid, vorzugsweise ein Gel, beaufschlagbar sind und durch vertikale bzw. horizontale Ausdehnung verschiedene Abstützmöglichkeiten für den Kopf des Benutzers bilden.

Zwei sich in radialer Richtung in Bezug auf die Achse Z gegenüberliegende Druckpolster 7 und 8 sind so gestaltet, dass sie beim Gebrauch jeweils außen an den Unterkieferbereichen des Benutzers anliegen. Die Druckpolster 7 und 8 besitzen vorzugsweise jeweils nur eine Druckkammer und Drücken den Kopf des Benutzers je nach den in ihnen aufgebauten Drücken nach rechts oder nach links um die Achse X.

An den Innenseiten der Druckpolster 7 und 8 sind sich ebenfalls gegenüberliegend zwei Druckpolster 9 und 10 angeordnet, die an den Unterseiten der seitlichen Unterkieferbereiche des Benutzers anliegen.

Um eine möglichst selektive Druckausübung auf die Unterkeiferbereiche zu ermöglichen, sind die Druckpolscer 9 und 10 vorzugsweise in der Umfangsrichtung gesehen jeweils durch eine innere Trennwand in ein vorderes Druckpolsterteil 91 bzw. 101 und ein hinteres Druckpolsterteil 92 bzw. 102 unterteilt, wobei die vorderen Druckpolsterteile 91 bzw. 101 an den Unterseiten der seitlichen vorderen Kieferbereiche anliegen und die hinteren Druckpolsterteile 92 bzw. 102 an den Unterseiten der seitlichen hinteren Kieferbereiche anliegen. An der Stelle von unterteilten Druckpolstern können auch getrennte Druckpolster nebeneinander angeordnet sein.

Die äußeren Druckpolster 7, 8 und 13, 14 gewährleisten den gleichbleibenden Anpreßdruck für die inneren Druckpolster 9, 10 bzw. 11, 12 an ihren Wirkstellen. Durch das dadurch erreichte stetige Anliegen der inneren Druckpolster 9, 10, 11, 12 werden somit auch Vibrationen wirksam gedämpft.

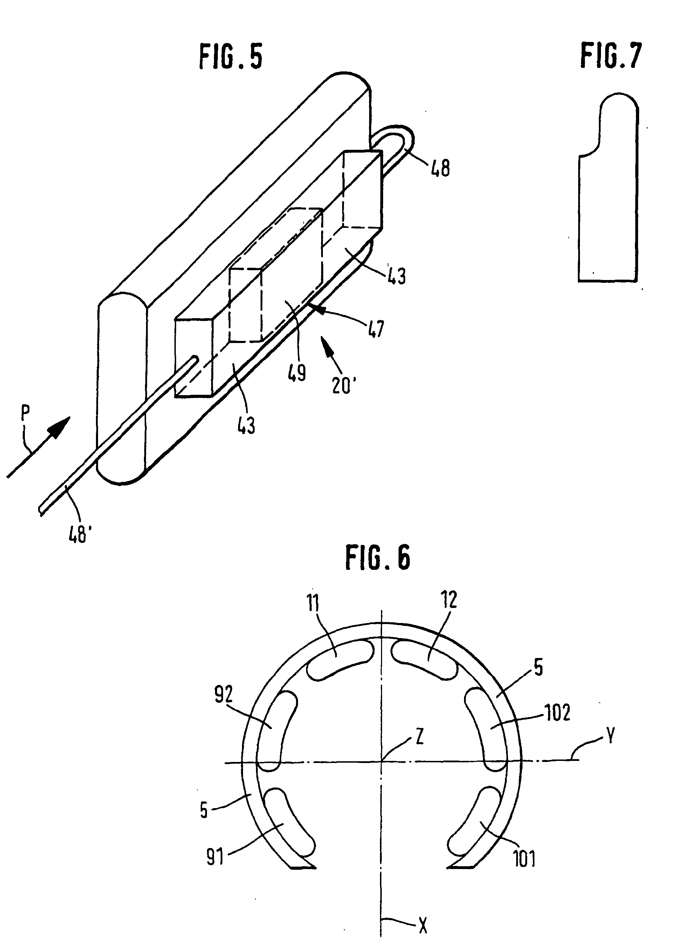

Gemäß der Figur 7 können die oberen Innenflächen der äußeren Druckpolster 7, 8, 13, 14 vorzugsweise entsprechend ihrer Anlagefläche anatomisch profiliert sein. Dabei ist eine dem Verlauf und der Form der Unterkieferäste bzw. der Hinterhauptschuppe angepaßte Anlagefläche gewährleistet.

Im hinteren Bereich, der dem Schlitz 22 gegenüberliegt, sind innen vorzugsweise zwei Druckpolster 11 und 12 und dahinter vorzugsweise zwei Druckpolster 13 und 14 angeordnet. Die Druckpolster 11 und 12 liegen oberseitig an dem Bereich des Kopfes des Benutzers an, an dem sich der Hinterkopf nach außen wölbt. Die Druckpolster 13 und 14 liegen außenseitig am Hinterkopf des Benutzers an. Durch Druckbeaufschlagung der Druckpolster 13 und 14 ist eine Druckausübung um die Achse Y möglich.

Die Druckpolster 7, 8, 13 und 14 bewirken eine Neigung des Kopfes um die X- oder Y-Achse. Hierbei stützen sie sich an dem Wandteil 5 ab. Dagegen dienen die Druckpolster 9, 10, 11 und 12 dazu, Kräfte in der Richtung der Z-Achse auszuüben. Hierzu stützen sie sich unterseitig an der Stützplatte 4 ab. In der Figur 2 und 3 sind die einzelnen oben genannten Kräfte dargestellt, und mit "K..." bezeichnet, wobei die jeweiligen Zahlen die Druckpolster bezeichnen, die diese Kräfte jeweils erzeugen. Der Einfachheit halber sind die einzelnen Druckpolster in den Figuren 2 und 3 nicht dargestellt.

Im folgenden werden die Funktion und die Ansteuerung der Druckpolster 7 bis 14 näher erläutert.

Die in der Richtung der Z-Achse Kräfte K9, K10, K11 und K12 entfaltenden Druckpolster 9, 10. 11 und 12 weisen an ihren oberen Flächen Sensoren S9, S10, S11 und S12 auf, die den auf ihre Kopf-Anlageflächen einwirkenden Druck jeweils anzeigen. In den Figuren sind diese Sensoren durch punktierte Flächen dargestellt.

Entsprechend weisen die Druckpolster 7, 8, 13 und 14 an ihren Innenflächen Sensoren S7, S8, S13 bzw. S14 (punktierte Flächen) auf, die den auf ihre Kopf-Anlageflächen einwirkenden Druck anzeigen.

Die Druckpolster 7, 8, 9, 10, 11, 12, 13, 14 sind zweckmäßigerweise so angeordnet, dass sie mit der jeweiligen Pilotenausstattung (z. B. Helm, Sauerstoffmaske) harmonisch kombinierbar sind.

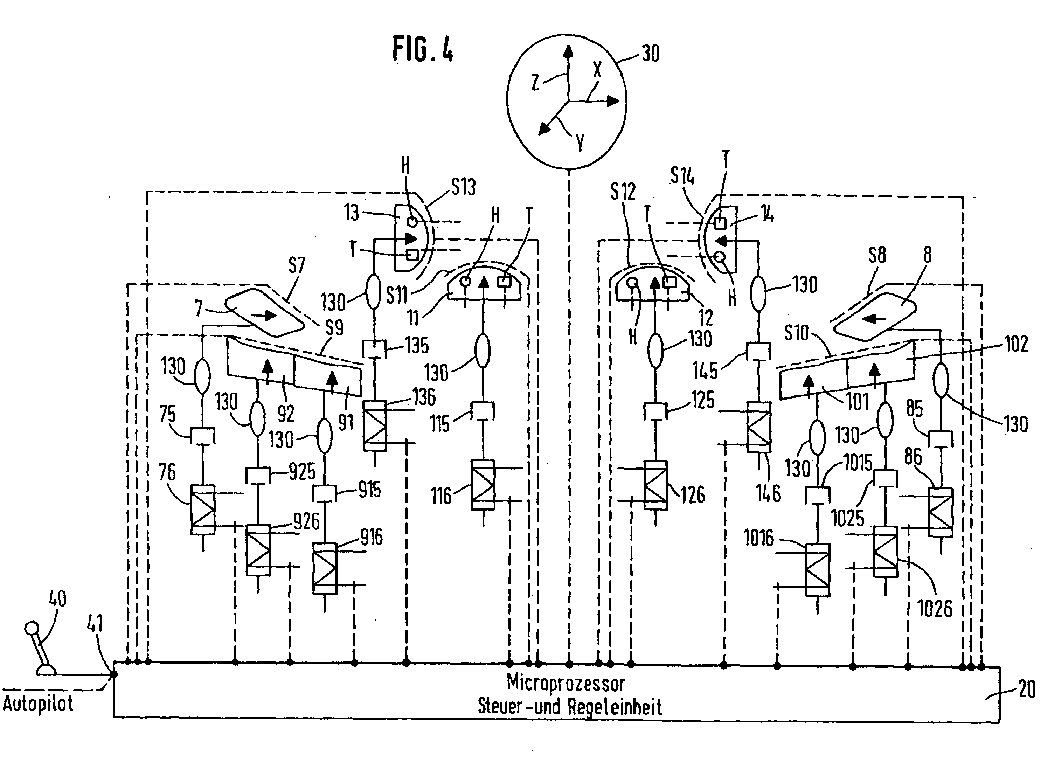

Gemäß Figur 4 ist jedes Druckpolster bzw. Druckpolsterteil über eine Druckleitung mit einem Druckgeber verbunden, der durch einen elektromechanischen Antrieb betätigbar ist. Auf diese Weise ist in jedem Druckpolster oder in jeder Druckkammer der Druck selektiv in der später erläuterten Weise einstellbar. Im einzelnen sind dem Druckpolster 7 ein Druckgeber 75 und ein Antrieb 76, dem Polsterteil 8 ein Druckgeber 85 und ein Antrieb 86, dem Druckpolsterteil 91 ein Druckgeber 915 und ein Antrieb 916, dem Druckpolsterteil 92 ein Druckgeber 925 und ein Antrieb 926, dem Druckpolsterteil 101 ein Druckgeber 105 und ein Antrieb 106, dem Druckpolsterteil 102 ein Druckgeber 1025 und Antrieb 1026, dem Polsterteil 11 ein Druckgeber 115 und ein Antrieb 116 dem Polsterteil 12 ein Druckgeber 125 und ein Antrieb 126, dem Polsterteil 13 ein Druckgeber 135 und ein Antrieb 136 und dem Polsterteil 14 ein Druckgeber 145 und ein Antrieb 146 zugeordnet. Jeder Antrieb ist mit einer Versorgungsquelle (nicht dargestellt) verbunden. Außerdem ist jeder Antrieb über eine Signalleitung in der in der Figur 4 dargestellten Weise mit einer Recheneinheit 20 verbunden, die vorzugsweise die Form eines Mikroprozessors aufweist.

In den einzelnen Druckpolstern bzw. Druckpolsterteilen, insbesondere in den Druckpolstern 11, 12, 13 und 14, die sich im Halswirbelbereich des Benutzers befinden, kann jeweils ein. Temperatursensor T und ein Kühl- und/oder Heizelement H angeordnet sein. Die Verbindungen der Temperatursensoren T und der Heiz- und oder Kühlelemente H mit der Steuereinheit 20 sind in der Figur 4 der Einfachheit halber nicht dargestellt. Auf diese Weise kann insbesondere der Halswirbelbereich eines Benutzers bedarfsweise erwärmt oder gekühlt werden.

Jeder Drucksensor S7, S8, S9, S10, S11, S12, S13, S14 ist über eine Leitung in der dargestellten Weise mit der Steuereinheit 20 verbunden.

Die Ansteuerung der Druckpolster bzw. Druckpolsterteile erfolgt in der folgenden Weise. Im normalen Betrieb wird eine unbehinderte Kopfbewegung des Benutzers dadurch ermöglicht, dass insbesondere in den sich seitlich gegenüberliegenden Druckpolstern 7 und 8 dann, wenn das eine Druckpolster, beispielsweise das Druckpolster 7, bei der Bewegung des Kopfes zu einer Seite, beispielsweise nach rechts, der Drucksensor S7 der Steuereinheit 20 einen erhöhten Druck anzeigt, der Antrieb 76 durch die Steuereinheit 20 erniedrigt und der Druck in dem Druckpolster S8 Betätigen des Antriebes 86 durch die Steuereinheit 20 um denselben Betrag erhöht. Die in den Druckpolstern 7 und 8 dabei erreichten Druckwerte werden beibehalten, bis der Benutzer den Kopf erneut in eine andere Position bewegt.

In der entsprechenden weise wird der Druck in den Druckpolstern 13 und 14 durch Betätigen der Druckgeber 135, 145 durch die Antriebe 136, 146 erniedrigt, wenn die Drucksensoren S13 und S14 anzeigen, dass der Benutzer durch Bewegen seines Kopfes nach hinten auf die Druckpolster 13, 14 einen Druck ausübt. Umgekehrt wird der Druck in den Polsterteilen 13 und 14 durch Betätigen der Druckgeber 135 bzw. 145 durch den Antrieb 136 bzw. 146 erhöht, wenn der Drucksensor S13 bzw. S14 anzeigt, dass der Druck in den Druckpolstern 13 bzw. 14 beim Bewegen des Kopfes nach vorne erniedrigt wird.

Ebenso erfolgt die Druckregelung in den Polstern 9, 10 bzw. in den Polsterteilen 91, 92, 101, 102 und in den Polstern 11,12 beim Drehen des Kopfes. Wenn ein Drucksensor S9 bzw. 310 auf einer Seite eine Druckerhöhung und der andere Drucksensor S10 oder S9 auf der anderen Seite eine Druckerniedrigung anzeigen, wird der Druck in dem zugehörigen Druckpolster durch Ansteuern des entsprechenden Druckgebers entsprechend erniedrigt und in dem anderen Polster entsprechend erhöht. Wenn der Drucksensor S11 oder S12 dabei eine Druckerhöhung und der Drucksensor S12 oder S11 eine Drukkerniedrigung anzeigen, wird der Druck durch Ansteuern des entsprechenden Druckgebers in dem Polster 11 oder 12 erhöht und in dem anderen Polster 12 oder 11 entsprechend erniedrigt.

Von wesentlicher Bedeutung ist es, dass bei der Einwirkung einer G-Kraft auf den Benutzer die Wirbelsäule, insbesondere die Halswirbelsäule desselben dadurch entlastet wird, dass die Druckpolster 9, bzw. 91, 92, 10 bzw. 101, 102, 11 und 12 eine der G-Kraft entgegenwirkende Druckkraft durch Betätigen der Druckgeber 915, 925, 1015, 1025 durch die Antriebe 916, 926, 1016, 1026, 116, 126 vergrößert. Dabei ermittelt die Steuereinheit 20 in Abhängigkeit von den ihr vom G-Sensor 30 zur Verfügung gestellten Werten der auf den Kopf betrags- und richtungsmäßig einwirkenden G-Kraftkomponenten die diesen Komponenten in den einzelnen Polstern bzw. Polsterteilen jeweils entgegenzusetzenden Drücke. Dies bedeutet, dass in den einzelnen genannten Druckpolstern bzw. Druckpolsterteilen je nach der Kopfneigung, die bei dem Einwirken der G-Kräfte gerade vorherrscht, unterschiedliche Gegenkräfte aufgebaut werden.

Gemäß Figur 4 ist es auch denkbar, vom sogenannten Autopiloten bzw. von dem vom Piloten betätigten Steuerstick Signale abzuleiten, die ein früheres Ansprechen der Einrichtung ermöglichen. Wenn der Steuerstick 80 betätigt wird, kann aufgrund der Art der Betätigung ein Signal an einen Eingang 41 der Stuer- und Regeleinheit 20 gegeben werden, das je nach Betätigungsrichtung und -stärke anzeigt, in welcher Richtung und Größe G-Kräfte zu erwarten sind. Dabei wird das genannte Signal von einem Rechner (nicht dargestellt) aufgrund der Art der Betätigung des Steuersticks 40 berechnet. Noch bevor die entsprechenden Druckpolster durch das vom G-Kraft-Sensor 30 erzeugte Signale betätigt werden, können somit die entsprechenden Druckgeber zur Beschleunigung des System betätigt und die entsprechenden Druckkammern vorab beaufschlagt werden, wobei die Endwerte der jeweiligen Drücke dann durch das vom G-Kraft-Sensor 30 ermittelte Signal eingestellt werden. Auf diese Weise kann das System erheblich beschleunigt werden. Bei der Steuerung mit einem Autopiloten kann das Signal am Eingang 41 von diesem Autopiloten abgeleitet werden. Es ist auch denkbar, dass anders ausgestaltete Schnittstellen, z.B. die Kreiselplattform eines Flugzeuges, zur Ableitung des Signales herangezogen werden.

Die einzelnen Druckpolster bestehen vorzugsweise aus Kunststoffkammern, in denen sich ein geeignetes Fluid, beispielsweise in der Form eines Gels, befindet. Bei den Drucksensoren handelt es sich um druckempfindliche Beschichtungen bestimmter Oberflächenbereiche der Druckpolster bzw. Druckpolsterteile (siehe Figur 1).

Um eine Ausdehnung der Druckpolster in der gewünschten Wirkrichtung, d.h. also vertikal oder horizontal zu gewährleisten, ist die Folie, aus denen die Druckpolster bzw. Druckpolsterteile hergestellt sind, vorzugsweise in der Wirkrichtung dicker gestaltet als die senkrecht zur Wirkrichtung verlaufenden Wände. Außerdem sind vorzugsweise halbelastische Stützen im Druckpolster quer zur Wirkrichtung vorgesehen, die die Seitenwände in einem definierten Abstand voneinander halten. Es wird darauf hingewiesen, dass die voranstehende Beschreibung der Druckpolster bzw. Druckpolsterteile lediglich beispielhaft ist und dass im Rahmen der vorliegenden Erfindung auch beliebig andere Ausführungsformen anwendbar sind, die gewährleisten, dass sich die Druckpolster bzw. Druckpolsterteile in der Wirkrichtung wesentlich stärker ausdehnen können als senkrecht zur Wirkrichtung.

Es wird darauf hingewiesen, dass bei besonders einfachen Ausführungsformen der vorliegenden Erfindung lediglich zum Stützen in der vertikalen Richtung dienende Druckpolster vorgesehen sein können. Solche einfachen Ausführungen eignen sich insbesondere für Bobfahrer und Skirennfahrer. Bei der Übung solcher Sportarten kommt es darauf an, die Halswirbelsäule der Sportler vor schädlichen Stößen und Belastungen zu schützen und den Kopf der Sportler möglichst in die für die spezielle Sportart typische und erwünschte Haltung zu bringen und in dieser Stellung zu halten. Bei diesen einfachen Ausführungsformen handelt es sich vorzugsweise um inaktive Versionen der erfindungsgemäßen Einrichtung, die nicht elektronisch steuerbar sind.

Vereinfachte aktive Versionen der erfindungsgemäßen Einrichtung können beispielweise neben der beschriebenen Anwendung bei Flugzeugen, insbesondere bei Düsenflugzeugen, auch auf dem Sportsektor, insbesondere beim Fahren von Rennfahrzeugen und Rennbooten Verwendung finden. Hier können die im Zusammenhang mit den Figuren 1 bis 3 beschriebenen Druckpolster bzw. Druckpolsterteile 9, 10, 11 und 12 entfallen, da es bei der Ausübung dieser Sportarten vor allem darauf ankommt, die Halswirbelsäule der Sportler vor vertikalen Belastungen, aber auch vor horizontalen und paraaxialen Beschleunigungskräften (HWS-Schleudertrauma oder "wiplash-Trauma") zu schützen.

Die vorliegende Erfindung läßt sich auch zur Entlastung der Halswirbelsäule von Motorradfahrern, Autofahrern, Bobfahrern, Skirennfahrern etc. verwenden. Dabei werden die Druckpolster zum bloßen Schutz und zur Stabilisierung schlagartig unter Druck gesetzt, wenn ein Schlag, eine Erschütterung oder eine Trennung des Menschen vom Fahrzeug oder Sportgerät eintritt. Die Triggerung erfolgt dabei nach der Art der Auslösung eines Airbags. Als Medium für die Druckpolster eignet sich neben einem Gel oder einer Flüssigkeit insbesondere ein Gas, das z.B. durch Zerstörung von CO₂-Kapseln bereitgestellt wird. Vorzugsweise werden dabei nur Druckpolster 9, 10, 11, 12 beaufschlagt, die in vertikaler Richtung wirken.

Eine weitere wichtige Anwendung der Erfindung liegt auf dem Gebiete des isometrischen und isokinetischen Auftrainierens der Halswirbelsäule. Der Trainierende oder ein Patient muß dabei den voreingestellten Drücken der Druckpolster in den verschiedenen Richtungen durch Druckausübung mit dem Kopf gezielt muskulär entgegenwirken. Dadurch können muskuläre Schwächen und Defizite wieder kompensiert werden oder es kann z.B. bei Piloten durch ein gezieltes Training ein muskuläres Stützkorsett zur Entlastung der Hals- und Nackenmuskulator antrainiert werden. Dabei werden die Druckpolster in individueller Abstimmung auf den muskulären Zustand des Trainierenden unter Druck gesetzt.

Dies erfolgt vorzugsweise wieder durch die erläuterte Steuer- und Regeleinrichtung 30, der zur Betätigung der Druckventile in der zur Therapie gewünschten Weise bestimmte Signale, beispielsweise am Eingang 41, eingegeben werden.

Aus der Figur 5 geht eine besonders einfach realisierbare Steuer- und Regeleinrichtung 20' hervor, die die Form einer jedem zu betätigenden Druckpolster zugeordneten Kol-Den/Zylinderanordnung 47 aufweist, die über eine Druckleitung 48 mit dem Druckpolster verbunden ist. Wirkt die G-Kraft in der Richtung des Pfeiles P, wird der Kolben 49 der Kolben/Zylinderanordnung 47 in der Richtung der G-Kraft P im Zylinderraum 43 der Kolben/Zylinderanordnung 47 verschoben, wobei im Zylinderraum 43 enthaltenes Fluid, vorzugsweise ein GE1, aus dem Zylinder 43 über die Leitung 48 in das Druckpolster gedrückt wird, sodass in diesen der Druck erhöht wird. Nach dem Ende der Einwirkung der G-Kraft wird der Kolben 49 durch die vom Kopf auf das Druckpolster ausgeübte Kraft wieder in Richtung auf seine Ausgangslage zurückgeführt, wobei der Druck im Druckpolster wieder abgebaut wird.

Wenn das jeweils gegenüberliegende Druckpolster über eine Leitung 48' mit dem Zylinderraum 43' an der dem Raum 43 gegenüberliegenden Seite des Kolbens 49 verbunden ist, wird bei einer Druckerhöhung im Druckpolster der Druck im gegenüberliegenden Druckpolster erniedrigt und umgekehrt.

Allgemein ausgedrückt sind gemäß Figur 6 bei der erfindungsgemäßen Einrichtung entlang des Umfanges vorzugsweise die Druckpolster 11, 12, 91, 92, 101, 102 angeordnet, die auch schon in der Figur 1 dargestellt sind, wobei die Druckpolster 91, 92 vorzugsweise zum Druckpolster 9 und die Druckpolster 101 und 102 vorzugsweise zum Druckpolster 10 zusammengefaßt sein können. Mit der Hilfe der Druckpolster und deren selektiver Druckbeaufschlagung durch die Steuerund Regeleinheit 20 ist eine Entlastung und Stützung der Halswirbelsäule bei schädlichen Belastungen möglich, wobei die Druckpolster je nach der Art der schädlichen Belastung selektiv durch die Steuer- und Regeleinheit 20 entsprechend einem Signal vom G-Kraft-Sensor 30, vom Autopiloten oder vom Steuerstick 41 beaufschlagt werden. Alternativ ist auch ein multivektorielles Training der Hals- und Nackenmuskulatur möglich, wobei die Steuer- und Regeleinheit 20 in individueller Abstimmung auf den muskulären Zustand des Trainierenden die Druckpolster selektiv unter Druck setzt. Durch ein solches Training kann erreicht werden, dass die trainierte Muskulatur im Belastungsfall schneller reagiert und auch sicherer hält, so dass Distorsionen bzw. Überdehnungen der Halswirbelsäule verhindert werden können.

Insbesondere bewirkt eine Druckbeaufschlagung der Druckpolster 11 und 12, dass der Kopf um die Y-Achse nach vorne geneigt wird.

Eine Druckbeaufschlagung der Druckpolster 91 und 101 bewirkt, dass der Kopf um die Y-Achse nach hinten geneigt wird.

Eine Druckbeaufschlagung der Polster 11, 12, 91 und 101 sowie gegebenenfalls 92 und 102 bewirkt, dass der Kopf in der Richtung der Z-Achse gestützt wird.

Eine Druckbeaufschlagung des Druckpolsters 102 sowie gegebenenfalls der Druckpolster 12 und 101 bewirkt, dass der Kopf in der Figur 6 um die Y-Achse nach links zur Seite geneigt wird.

Eine Druckbeaufschlagung des Druckpolsters 92 und gegebenenfalls der Druckpolster 11 und 91 bewirkt, dass der Kopf um die X-Achse nach rechts verschwenkt wird.

Durch unterschiedliche Druckbeaufschlagungen der Druckpolster 12, 102 und 101 oder der Druckpolster 11, 91 und 92 bewirkt, dass der Kopf je nach Art der Druckbeaufschlagung in beliebige Richtungen der X-Y-Ebene, d.h. also beispielsweise nach links unten, nach links oben, nach rechts oben oder nach rechts unten verschwenkt wird.

Je nach Art der Druckbeaufschlagung ist also eine Entlastung des Kopfes und eine Fixierung desselben bei schädlichen Belastungen in beliebigen Raumrichtungen möglich. Entsprechend können beim Trainieren mit der vorliegenden Einrichtung durch die genannten Druckbelastungen der Druckpolster in bestimmten Raumrichtungen zum speziellen Muskeltraining Gegendrücke aufgebaut werden, gegen die der Trainierende isometrisch und/oder isokinetisch und/oder multivektoriell und/oder zeitabhängig "Arbeiten" muß.

Es wird darauf hingewiesen, dass je nach der zu erzielenden Stütz- bzw. Trainingswirkung auch andere Druckpolsterzahlen und -kombinationen möglich sind. Die Druckpolster 11 und 12 bzw. 13 und 14 können auch entsprecehend den Druckpolstern 91, 92 bzw. 101, 102 zu einer Einheit zusammengefasst sein.

## Patentansprüche

1. Einrichtung zum Stützen der Halswirbelsäule, insbesondere beim Fahren in Fahrzeugen und insbesondere beim Fliegen mit Flugzeugen und beim Ausschuß aus denselben, mit einer den Hals eines Benutzers wenigstens teilweise umgebenden Druckpolstereinrichtung (9 bis 12), die sich mit einer Seite an der Schulter des Benutzers abstützt, wobei die Druckpolstereinrichtung (9 bis 12) sich in Bezug auf den Hals des Benutzers gegenüber liegend wenigstens ein erstes Druckpolster (9) und ein zweites Druckpolster (10), die sich mit ihren jeweils anderen Seiten an den Unterseiten der seitlichen Unterkieferbereiche des Benutzers abstützen, und wenigstens ein drittes Druckpolster (11) umfaßt, das im hinteren Bereich der Einrichtung angeordnet ist und sich mit seiner anderen Seite an der Hinterhauptschuppe des Benutzers abstützt, wobei das erste (9), zweite (10) und dritte Druckpolster (11) in ihrer Wirkrichtung einen Druck zur Abstützung des Kopfes in vertikaler Richtung bewirken, **dadurch gekennzeichnet, dass** eine Steuer- und Regeleinheit (20) vorgesehen ist, die den Druck in dem ersten (9), zweiten (10) und dritten (11) Druckpolster selektiv erhöhen oder erniedrigen kann, dass eine Vorrichtung (30) vorgesehen ist, die die auf den Kopf des Benutzers einwirkenden G-Kraftkomponenten in den drei Achsen ermittelt und dass die Steuer- und Regeleinheit (20) in dem ersten Druckpolster (9), dem zweiten Druckpolster (10) und dem dritten Druckpolster (11) je nach Kopfneigung, die beim Einwirken der G-Kraftkomponente gerade vorherrscht, unterschiedliche Gegenkräfte in der Wirkrichtung aufbaut.

2. Einrichtung nach Anspruch 1, **dadurch gekennzeichnet, dass** neben dem dritten Druckpolster (11) ein viertes Druckpolster (12) angeordnet ist, das sich mit seiner anderen Seite an der Hinterhauptschuppe des Benutzers abstützt, eine Abstützung des Kopfes in vertikaler Richtung bewirkt und durch die Steuerund Regeleinrichtung (20) selektiv ansteuerbar ist, um den Druck im vierten Druckpolster (12) wahlweise zu erhöhen oder zu erniedrigen.

3. Einrichtung nach Anspruch 1 oder 2, **dadurch gekennzeichnet, dass** das erste, zweite, dritte und gegebenenfalls das vierte Druckpolster (9, 10, 11, 12) sich mit 'ihrer einen Seite an einer Stützplatte (4) abstützen, die den Hals des Benutzers wenigstens teilweise ringförmig umgibt und sich auf dem Schultergürtel des Benutzers abstützt.

4. Einrichtung nach einem der Ansprüche 1 bis 3, **dadurch gekennzeichnet, dass** eine weitere Druckpolstereinrichtung (7, 8, 13, 14) vorgesehen ist, die einen Druck in einer weiteren Wirkrichtung erzeugt, die senkrecht zur ersten Wirkrichtung verläuft und ein fünftes Druckpolster (7) und ein sechstes Druckpolster (8) umfaßt, die sich in Bezug auf den Hals des Benutzers gegenüberliegen, wobei das fünfte Druckpolster (7) und das sechste Druckpolster (8) innenseitig an den äußeren Unterkieferbereichen des Benutzers anliegen.

5. Einrichtung nach Anspruch 4, **dadurch gekennzeichnet, dass** im hinteren Bereich der Einrichtung wenigstens ein siebtes Druckpolster (13), vorzugsweise ein siebtes Druckpolster (13) und ein achtes Druckpolster (14), der weiteren Druckpolstereinrichtung angeordnet sind, die innenseitig am äußeren Hinterkopf des Benutzers anliegen.

6. Einrichtung nach Anspruch 4 oder 5, **dadurch gekennzeichnet dass** an der Stützplatte (4) ein senkrecht zur Stützplatte (4) verlaufendes vertikales Wandteil (5) angeordnet ist, an dem sich das fünfte (7), das sechste (8) und/oder das siebte (11) und gegebenenfalls das achte (14) Druckpolster außenseitig abstützen.

7. Einrichtung nach einem der Ansprüche 1 bis 6, **dadurch gekennzeichnet, dass** die Stützplatte (4) außenseitig einen Randbereich (3) aufweist, der in einem Ringelement (2) einer Stützschale (1) drehbar geführt ist, wobei die Stützschale (1) so geformt ist, dass sie an den Schultergürtel des Benutzers individuell angepaßt ist, um sich auf diesem abstützen zu können.

8. Einrichtung nach Anspruch 7, **dadurch gekennzeichnet, dass** die Stützplatte (4), der Randbereich (3), das Ringelement (2) und die Stützschale (1) in ihrem vorderen Bereich eine Aussparung (22) aufweisen, durch die beim Aufweiten der Stützschale (1), des Ringelementes (2), des Randbereiches (3) und der Stützplatte (4) der Hals des Benutzers durchführbar ist.

9. Einrichtung nach Anspruch 8, **dadurch gekennzeichnet, dass** Verschließeinrichtungen (6) vorgesehen sind, die an Randbereichen (61) der Aussparung (22) der Stützschale (1) angreifen und bei deren Betätigung die Randbereiche (61) zur Befestigung der Einrichtung am Benutzer aufeinander zuziehbar sind.

10. Einrichtung nach Anspruch 9, **dadurch gekennzeichnet, dass** die Verschließeinrichtungen (6) die Form von Gurten oder Klammern aufweisen.

11. Einrichtung nach einem der Ansprüche 1 bis 10, **dadurch gekennzeichnet, dass** das erste Druckpolster (9), das zweite Druckpolster (10), das dritte Druckpolster (11) und gegebenenfalls das vierte Druckpolster (12) oberseitig Sensoren (S9, S10, S11, S12) aufweisen, die den auf ihre Kopf-Auflageflächen einwirkenden Druck jeweils anzeigen.

12. Einrichtung nach einem der Ansprüche 1 bis 11, **dadurch gekennzeichnet, dass** das fünfte Druckpolster (7), das sechste Druckpolster (8), das siebte Druckpolster (13) und gegebenenfalls das achte Druckpolster (14) an ihren Innenflächen Sensoren (S7, S8, S13, S14) aufweisen, die den auf ihre Kopf-Anlageflächen einwirkenden Druck jeweils anzeigen.

13. Einrichtung nach Anspruch 11 oder 12, **dadurch gekennzeichnet, dass** die Drucksensoren (S7, S8, S9, S10, S11, S12, S13, S14) jeweils mit einem durch einen Antrieb ansteuerbaren Druckregler verbunden sind, der den Druck im Druckpolster durch die Steuer- und Regeleinheit (20) gesteuert selektiv in der Wirkrichtung bzw. in der weiteren Wirkrichtung erhöhen oder erniedrigen kann, sodass bei der Verwendung der Vorrichtung zum Stützen der Halswirbelsäure der Kopf automatisch durch ein spezielles Druck- und Ausdehnungsmuster der Druckpolster in eine anatomisch günstige Sicherheitsposition gebracht wird.

14. Einrichtung nach einem der Ansprüche 1 bis 13, **dadurch gekennzeichnet, dass** das erste Druckpolster (9) und das zweite Druckpolster (10) durch eine vertikale Trennwand in zwei in der Umfangsrichtung nebeneinanderliegende Druckpolsterteile (91, 92 bzw. 101, 102) unterteilt sind, wobei jedem Druckpolsterteil (91, 92, 101, 102) ein eigener Druckgeber (915, 925, 1015, 1025) und ein eigener Antrieb (916, 926, 1016, 1026) zugeordnet sind.

15. Einrichtung nach Anspruch 13 oder 14, **dadurch gekennzeichnet, dass** zwischen den Druckpolstern bzw. den Druckpolsterteilen und dem Druckgeber jeweils ein Druckausgleichsbehältnis (130) angeordnet ist.

16. Einrichtung nach einem der Ansprüche 13 bis 14, **dadurch gekennzeichnet, dass** die Steuer- und Regeleinheit (20) zur Ermöglichung einer unbehinderten Kopfbewegung des Benutzers den Druck in dem fünften Druckpolster (7) und dem gegenüberliegenden sechsten Druckpolster (8) so steuert, dass bei der Bewegung des Kopfes zu einer Seite und Anzeige dieser Bewegung durch die dem fünften und sechsten Druckpolster zugeordneten Drucksensoren (S9, S10) der Druckgeber, der dem Druckpolster mit dem erhöhten Druck zugeordnet ist, den Druck um einen Betrag erniedrigt und der Druckgeber, der dem Druckpolster zugeordnet ist, in dem Druck erniedrigt wird, den Druck um denselben Betrag erhöht, und dass gegebenenfalls der Druck in dem siebten Druckpolster (13) und dem achten Druckpolster (14) durch Betätigen der Druckgeber (135, 145) durch die Antriebe (136, 146) erniedrigt wird, wenn die Drucksensoren (S13 und S14) anzeigen, dass der Bennutzer durch Bewegen seines Kopfes nach hinten auf diese siebsten und achten Druckpolster (13, 14) einen Druck ausübt und erhöht wird, wenn die Drucksensoren (S13, S14) anzeigen, dass der Druck in diesen siebten und achten Druckpolstern (513, S14) beim Bewegen des Kopfes nach vorne erniedrigt wird.

17. Einrichtung nach einem der Ansprüche 13 bis 16, **dadurch gekennzeichnet, dass** die Steuer- und Regeleinheit (20) in dem ersten Druckpolster (9) bzw. den ersten Druckpolsterteilen (91, 92), dem zweiten Druckpolster (10) bzw. den zweiten Druckpolsterteilen (101, 102), dem dritten Druckpolster (11) und gegebenenfalls dem vierten Druckpolster (12) durch Betätigen der Druckgeber (915, 925, 1015, 1025, 115, 125) durch die Antriebe (916, 926, 1016, 1026, 116, 126) bzw. in dem fünften Druckpolster (7), dem sechsten Druckpolster (8), dem siebten Druckpolster (13) und gegebenenfalls dem achten Druckpolster (14) durch Betätigen der Druckgeber (75, 85, 135, 145) durch die Antriebe (76, 86, 136, 146) je nach Kopfneigung die bei dem Einwirken der G-Kraftkomponenten gerade vorherrscht, unterschiedliche Gegenkräfte in der Wirkrichtung bzw. in der weiteren Wirkrichtung aufbaut.

18. Einrichtung nach einem der Ansprüche 1 bis 17, **dadurch gekennzeichnet, dass** die Druckpolster bzw. die Druckpolsterteile durch die Beschaffenheit ihrer Wandteile sich im wesentlichen in der Wirkrichtung bzw. in der weiteren Wirkrichtung ausdehnen können, wenn sie durch ein Fluid belastet werden und sich zusammenziehen können, wenn sie entlastet werden.

19. Einrichtung nach Anspruch 18, **dadurch gekennzeichnet, dass** zwischen den in der Wirkrichtung bzw. in der weiteren Wirkrichtung verlaufenden Wandteilen halbelastische Stützelemente vorgesehen sind.

20. Einrichtung nach einem der Ansprüche 1 bis 19, **dadurch gekennzeichnet, dass** die Druckpolster und gegebenenfalls die Druckausgleichsbehältnisse (130) mit einem Fluid, vorzugsweise einer gelartigen Flüssiakeit, gefüllt sind.

21. Einrichtung nach einem der Ansprüche 1 bis 20, **dadurch gekennzeichnet, dass** in Druckpolstern bzw. Druckpolsterteilen Heiz- und/oder Kühleinrichtungen (S, T) zur Fluiderwärmung bzw. -abkühlung vorgesehen sind.

22. Einrichtung nach einem der Ansprüche 2 bis 21, **dadurch gekennzeichnet, dass** die Stützplatte (4) mit einer auf den Schulterbereichen des Benutzers auflegbaren Stützschale (1) verbunden ist.

23. Einrichtung nach einem der Ansprüche 2 bis 22, **dadurch gekennzeichnet, dass** an der Stützschale (1) eine Verbindungsvorrichtung (46) zum dichten Verbinden der Stützschale (1) mit dem oberen Halsausschnitt eines Ganzkörperschutzanzuges vorgesehen ist.

24. Einrichtung nach Anspruch 23, **dadurch gekennzeichnet** 1 dass die Verbindungsvorrichtung (46) die Form einer dehnbaren, ringförmigen Dichtungseinrichtung aufweist.

25. Einrichtung nach Anspruch 24, **dadurch gekennzeichnet dass** die Verbindungseinrichtung (46) neben dem Ringelement (2) angeordnet ist.

26. Einrichtung nach einem der Ansprüche 1 bis 25, **dadurch gekennzeichnet, dass** den Druckpolstern Kolben/Zylinderanordnungen (47) als Steuer- und Regeleinheit zugeordnet sind, deren Kolben (49) bei einer Beaufschlagung in einer Richtung ein Fluid aus dem Zyliaderaum (43) in das Druckpolster drücken.

27. Einrichtung nach Anspruch 26, **dadurch gekennzeichnet, dass** die Kolben/Zylinderanordnungen außenseitig am Druckpolster oder an einem davon entfernten Ort angeordnet sind.

28. Einrichtung nach einem der Ansprüche 17 bis 27, **dadurch gekennzeichnet, dass** die Vorrichtung ein G-Kraft-Sensor (30), ein Steuerstick (40), eine Kreiselplattform oder ein Autopilot ist, die jeweils ein die G-Kraftkomponenten anzeigendes Signal erzeugen.

29. Einrichtung nach einem der Ansprüche 4 bis 28, **dadurch gekennzeichnet, dass** die Druckpolster der weiteren Druckpolstereinrichtung an ihren inneren Anlageflächen entsprechend dem Verlauf und der Form der Unterkieferäste bzw. der Hinterhauptschuppe ausgestaltet sind.

30. Anwendung der Einrichtung nach einem der Ansprüche 1 bis 29 auf dem Gebiete des Fliegens mit Düsenflugzeugen und beim Ausschuß aus denselben, des Kunstfluges und des Auto- und Bootrennsportes.

## Claims

1. An apparatus for supporting the cervical vertebral column, in particular when travelling in vehicles and in particular when flying in aeroplanes and when ejecting from them, having a pressure pad device (9 to 12) at least partially surrounding the user's neck, which is supported by one side on the user's shoulders, wherein the pressure pad device (9 to 12), positioned opposite the user's neck, comprises at least a first pressure pad (9) and a second pressure pad (10), which are supported by their respective other sides on the under sides of the lateral lower jaw areas of the user, and at least a third pressure pad (11), which is disposed in the rear region of the apparatus and is supported by its other side at the squamous part of the occipital bone of the user, wherein the first (9), the second (10) and third pressure pad (11) in their effective direction produce pressure to support the head in the vertical direction,
**characterised in that** a control and regulating unit (20) is provided, which can selectively increase or reduce the pressure in the first (9), second (10) and third (11) pressure pads,
**in that** a device (30) is provided which determines the G force components acting on the user's head in the three axes
and **in that** the control and regulating unit (20) builds up different counter-forces in the effective direction in the first pressure pad (9), the second pressure pad (10) and the third pressure pad (11), depending on the head inclination that prevails when the G force components are acting.

2. An apparatus according to Claim 1,
**characterised in that** next to the third pressure pad (11) is disposed a fourth pressure pad (12), which is supported by its other side on the squamous part of the occipital bone of the user, produces support for the head in the vertical direction and can be selectively controlled by the control and regulating device (20) in order selectively to increase or reduce the pressure in the fourth pressure pad (12).

3. An apparatus according to Claim 1 or 2,
**characterised in that** the first, second, third and possibly the fourth pressure pads (9, 10, 11, 12) are supported by their one side on a support plate (4), which surrounds the user's neck at least partially in a circle and is supported on the user's shoulder girdle.

4. An apparatus according to one of Claims 1 to 3,
**characterised in that** another pressure pad device (7, 8, 13, 14) is provided, which produces pressure in another effective direction which extends perpendicularly to the first effective direction and comprises a fifth pressure pad (7) and a sixth pressure pad (8), which are situated opposite the user's neck, the fifth pressure pad (7) and the sixth pressure pad (8) being adjacent to the external lower jaw areas of the user.

5. An apparatus according to Claim 4,
**characterised in that** disposed in the rear region of the apparatus are at least a seventh pressure pad (13), preferably a seventh pressure pad (13) and an eighth pressure pad (14), of the other pressure pad device, which on the inside are adjacent to the outer rear head of the user.

6. An apparatus according to Claim 4 or 5,
**characterised in that** disposed on the support plate (4) is a vertical wall part (5) extending perpendicularly to the support plate (4), against which the fifth (7), the sixth (8) and/or the seventh (11) and possibly the eighth (14) pressure pads are supported on the outside.

7. An apparatus according to one of Claims 1 to 6,
**characterised in that** the support plate (4) comprises on its outside an edge region (3), which is rotatably guided in a ring element (2) of a support shell (1), the support shell (1) being formed so that it can be individually adapted to the user's shoulder girdle so that it can be supported thereon.

8. An apparatus according to Claim 7,
**characterised in that** the support plate (4), the edge region (3), the ring element (2) and the support shell (1) comprise a recess (22) in their front region, through which the user's neck can be passed when the support shell (1), the ring element (2), the edge region (3) and the support plate (4) are expanded.

9. An apparatus according to Claim 8,
**characterised in that** locking devices (6) are provided, which act on edge regions (61) of the recess (22) of the support shell (1) and upon their actuation the edge regions (61) can be pulled tight towards one another to fasten the apparatus on the user.

10. An apparatus according to Claim 9,
**characterised in that** the locking devices (6) take the form of straps or clips.

11. An apparatus according to one of the preceding Claims,
**characterised in that** the first pressure pad (9), the second pressure pad (10) the third pressure pad (11) and possibly the fourth pressure pad (12) comprise sensors (S9, S10, S11, S12) on their top side, which in each case indicate the pressure acting on their head bearing surfaces.

12. An apparatus according to one of Claims 1 to 11,
**characterised in that** the fifth pressure pad (7), the sixth pressure pad (8), the seventh pressure pad (13) and possibly the eighth pressure pad (14) comprises sensors (S7, S8, S13, S14) on their inner surfaces, which indicate the pressure acting in each case on their head bearing surfaces.

13. An apparatus according to Claim 11 or 12,
**characterised in that** the pressure sensors (S7, S8, S9, S10, S11, S12, S13, S14) are each connected to a pressure regulator, which can be controlled by a drive, and which can increase or reduce the pressure in the pressure pad controlled selectively by the control and regulating unit (20) in the effective direction or in the other effective direction so that when the apparatus is used to support the cervical vertebral column the head is automatically brought into an anatomically favourable safety position by a special pressure and expansion patterns of the pressure pads.

14. An apparatus according to one of Claims 1 to 13,
**characterised in that** the first pressure pad (9) and the second pressure pad (10) are divided by a vertical partition into two pressure pad parts (91, 92 and 101, 102) adjacent to one another n the circumferential direction, each pressure pad part (91, 92, 101, 102) being assigned its own pressure transducer (915, 925, 1015, 1025) and its own drive (916, 926, 1016, 1026).

15. An apparatus according to Claim 13 or 14,
**characterised in that** a pressure compensation case (130) is disposed between the pressure pads or the pressure pad parts and the pressure transducer in each case.

16. An apparatus according to one of Claims 13 to 14,
**characterised in that** to enable an unimpeded head movement by the user, the control and regulating unit (20) controls the pressure in the fifth pressure pad (7) and the opposite sixth pressure pad (8) so that, when the head is moved to one side and this movement is indicated by the pressure sensors (S9, S10) associated with the first and sixth pressure pads, the pressure transducer associated with the pressure pad with the increased pressure reduces the pressure by an amount, and the pressure transducer associated with the pressure pad in which pressure is reduced increases the pressure by the same amount,
and **in that** possibly the pressure in the seventh pressure pad (13) and the eighth pressure pad (18) is reduced by actuating the pressure transducers (135, 145) by the drives (136, 146) if the pressure sensors (S13 and S14) indicate that the user by moving his head backwards exerts a pressure on these seventh and eight pressure pads (13, 14), and is increased when the pressure sensors (S13, S14) indicate that the pressure in these seventh and eighth pressure pads (S13, S14) is reduced when the head is moved forwards.

17. An apparatus according to one of Claims 13 to 16,
**characterised in that** the control and regulating unit (20) builds up different counter forces in the effective direction or in the other effective direction in the first pressure pad (9) or the first pressure pad parts (91, 92), the second pressure pad (10) or the second pressure pad parts (101, 102), the third pressure pad (11) and possibly the fourth pressure pad (12) by actuating the pressure transducers (915, 925, 1015, 1025, 115, 125) by the drives (916, 926, 1016, 1026, 116, 126) or in the fifth pressure pad (7), the sixth pressure pad (8), the seventh pressure pad (13) and possibly the eight pressure pad (145) by actuating the pressure transducers (75, 85, 135, 145) by the drives (76, 86, 136, 146) depending on the head inclination which prevails during the action of the G force components.

18. An apparatus according to one of Claims 1 to 17,
**characterised in that** the pressure pads or the pressure pad parts can, as a result of the nature of their wall parts, expand substantially in the effective direction or in the other effective direction when they are acted upon by a fluid and can contract when they are relieved.

19. An apparatus according to Claim 18,
**characterised in that** semi-elastic support elements are provided between the wall parts extending in the effective direction or in the other effective direction.

20. An apparatus according to one of Claims 1 to 19,
**characterised in that** the pressure pads and possibly the pressure compensation cases (130) are filled with a fluid, preferably a gel-type liquid.

21. An apparatus according to one of Claims 1 to 20,
**characterised in that** heating and/or cooling devices (S, T) are provided for fluid heating and cooling in pressure pads or pressure pad parts.

22. An apparatus according to one of Claims 2 to 21,
**characterised in that** the support plate (4) is connected to a support shell (1) which can be laid on the shoulder areas of the user.

23. An apparatus according to one of Claims 2 to 22,
**characterised in that** at the support shell (1) a connecting device (46) is provided for tightly connecting the support shell (1) to the upper neck opening of a whole-body protective suit.

24. An apparatus according to Claim 23,
**characterised in that** the connecting device (46) takes the form of an expandable, annular sealing device.

25. An apparatus according to Claim 24,
**characterised in that** the connecting device (46) is disposed next to the ring element (2).

26. An apparatus according to one of Claims 1 to 25,
**characterised in that** associated with the pressure pads are piston/cylinder arrangements (47) as the control and regulating unit, the pistons (49) of which force a fluid out of the cylinder chamber (43) into the pressure pad when acted upon in one direction.

27. An apparatus according to Claim 26,
**characterised in that** the piston/cylinder arrangements are disposed on the outside of the pressure pad or at a site remote therefrom.

28. An apparatus according to one of Claims 17 to 27,
**characterised in that** the device is a G force sensor (30), a control stick (40), a gyroplatform or an autopilot, which in each case produce a signal indicating the G force components.

29. An apparatus according to one of Claims 4 to 28,
**characterised in that** the pressure pads of the other pressure pad device are configured at their inner bearing surfaces according to the path and the shape of the ramie of the lower jaw and the squamous part of the occipital bone.

30. Application of the apparatus according to one of Claims 1 to 29 in the field of flying with jet aeroplanes and when ejecting therefrom, in the field of stunt flight and boat racing.

## Revendications

1. Dispositif de maintien de la colonne cervicale, notamment lors de la conduite de véhicules automobiles ainsi que notamment lors de vols en avion et lors de l'éjection hors de ceux-ci, avec un système de coussins sous pression (9 à 12) qui enserre au moins partiellement le cou d'un utilisateur et prend appui d'un côté sur l'épaule de l'utilisateur, le système de coussins sous pression (9 à 12) comprenant, disposés en vis-à-vis par rapport au cou de l'utilisateur, au moins un premier coussin sous pression (9) et un deuxième coussin sous pression (10), qui prennent appui par leur autre côté sur la surface inférieure des régions latérales du maxillaire inférieur de l'utilisateur, et au moins un troisième coussin sous pression (11) qui est disposé dans la région postérieure du dispositif et prend appui par son autre côté sur l'os occipital de l'utilisateur, le premier (9), le deuxième (10) et le troisième (11) coussin sous pression produisant dans leur direction d'action une poussée pour le maintien de la tête dans la direction verticale, **caractérisé par le fait qu'**il est prévu une unité de commande et de réglage (20) qui peut augmenter ou abaisser de manière sélective la pression dans le premier (9), le deuxième (10) et le troisième (11) coussin sous pression, **par le fait qu'**il est prévu un dispositif (30) qui détermine les composantes de force d'accélération G agissant sur la tête de l'utilisateur suivant les trois axes, et **par le fait que** l'unité de commande et de réglage (20) établit dans le premier (9), le deuxième (10) et le troisième (11) coussins sous pression, dans la direction d'action, des forces antagonistes variables en fonction de l'inclinaison de la tête résultant de l'action de la composante de force d'accélération G.

2. Dispositif selon la revendication 1, **caractérisé par le fait qu'**à côté du troisième coussin sous pression (11) est disposé un quatrième coussin sous pression (12), qui prend appui avec son autre côté contre l'os occipital de l'utilisateur, assure un maintien de la tête dans la direction verticale et peut être commandé de manière sélective par le dispositif de commande et de réglage (20) aux fins d'augmenter ou d'abaisser la pression dans ledit quatrième coussin sous pression (12).

3. Dispositif selon la revendication 1 ou 2, **caractérisé par le fait que** les premier, deuxième, troisième et le cas échéant quatrième coussins sous pression (9, 10, 11, 12) prennent appui d'un côté sur une plaque de support (4) qui entoure au moins partiellement, en formant un anneau, le cou de l'utilisateur et repose sur la ceinture scapulaire de l'utilisateur.

4. Dispositif selon une des revendications 1 à 3, **caractérisé par le fait qu'**il est prévu un système de coussins sous pression (7, 8, 13, 14) supplémentaire, qui produit une poussée dans une direction d'action supplémentaire, perpendiculaire à la première direction d'action, et qui comprend un cinquième coussin sous pression (7) et un sixième coussin sous pression (8) disposés en vis-à-vis par rapport au cou de l'utilisateur, le cinquième coussin sous pression (7) et le sixième coussin sous pression (8) étant en contact côté intérieur avec les régions extérieures du maxillaire inférieur de l'utilisateur.

5. Dispositif selon la revendication 4, **caractérisé par le fait que** dans la zone postérieure du dispositif est disposé au moins un septième coussin sous pression (13), de préférence un septième coussin sous pression (13) et un huitième coussin sous pression (14) du système de coussins sous pression supplémentaire, qui sont en contact côté intérieur avec l'extérieur de l'os occipital de l'utilisateur.

6. Dispositif selon la revendication 4 ou 5, **caractérisé par le fait que** sur la plaque de support (4) est disposé un élément de paroi (5) vertical, perpendiculaire à la plaque de support (4), sur lequel le cinquième (7), le sixième (8) et/ou le septième (11) et le cas échéant le huitième (14) coussin sous pression prennent appui côté extérieur.

7. Dispositif selon une des revendications 1 à 6, **caractérisé par le fait que** la plaque de support (4) présente côté extérieur une partie de bord (3) qui est guidée avec possibilité de rotation dans un élément annulaire (2) d'une coque de support (1), la coque de support (1) étant conformée de manière à pouvoir être adaptée individuellement à la ceinture scapulaire de l'utilisateur aux fins de prendre appui sur celle-ci.

8. Dispositif selon la revendication 7, **caractérisé par le fait que** la plaque de support (4), la partie de bord (3), l'élément annulaire (2) et la coque de support (1) comportent dans leur région antérieure une découpe (22), par laquelle le cou de l'utilisateur peut passer lorsqu'on écarte la coque de support (1), l'élément annulaire (2), la partie de bord (3) et la plaque de support (4).

9. Dispositif selon la revendication 8, **caractérisé par le fait qu'**il est prévu des dispositifs de fermeture (6) qui agissent sur les bords (61) de la découpe (22) de la coque de support (1) et dont l'actionnement permet de rapprocher les bords (61) aux fins de fixer le dispositif sur l'utilisateur.

10. Dispositif selon, la revendication 9, **caractérisé par le fait que** les dispositifs de fermeture (6) se présentent sous la forme de sangles ou d'agrafes.

11. Dispositif selon une des revendications 1 à 10, **caractérisé par le fait que** le premier coussin sous pression (9), le deuxième coussin sous pression (10), le troisième coussin sous pression (11) et le cas échéant le quatrième coussin sous pression (12) présentent côté supérieur des détecteurs (S9, S10, S11, S12) qui indiquent la pression agissant sur leur surface d'appui de la tête.

12. Dispositif selon une des revendications 1 à 11, **caractérisé par le fait que** le cinquième coussin sous pression (7), le sixième coussin sous pression (8), le septième coussin sous pression (13) et le cas échéant le huitième coussin sous pression (14) présentent sur leur surface intérieure des détecteurs (S7, S8, S13, S14) qui indiquent la pression agissant sur leur surface d'appui de la tête.

13. Dispositif selon la revendication 11 ou 12, **caractérisé par le fait que** les détecteurs de pression (S7, S8, S9, S10, S11, S12, S13, S14) sont reliés à un moyen de réglage de pression actionné par un mécanisme d'entraînement, qui peut augmenter ou abaisser de manière sélective, contrôlée par l'unité de commande et de réglage (20), dans l'une ou dans l'autre direction d'action, la pression dans le coussin sous pression, de telle sorte que lors de l'utilisation du dispositif de maintien de la colonne cervicale, la tête soit amenée automatiquement par un modèle spécial de pression et expansion des coussins sous pression, dans une position favorable du point de vue anatomique.

14. Dispositif selon une des revendications 1 à 13, **caractérisé par le fait que** le premier coussin sous pression (9) et le deuxième coussin sous pression (10) sont divisés par une cloison verticale en deux parties de coussin sous pression (91, 92 et 101, 102) contiguës dans la direction périphérique, un capteur de pression (915, 925, 1015, 1025) propre et un mécanisme d'entraînement (916, 926, 1016, 1026) propre étant associész à chaque partie de coussin sous pression (91, 92, 101, 102).

15. Dispositif selon la revendication 13 ou 14, **caractérisé par le fait qu'**entre les coussins sous pression ou les parties de coussin sous pression et le capteur de pression est disposé chaque fois un volume d'équilibrage de pression (130).

16. Dispositif selon une des revendications 13 à 14, **caractérisé par le fait que** l'unité de commande et de réglage (20), afin de permettre des mouvements sans entrave de la tête de l'utilisateur, contrôle la pression dans le cinquième coussin sous pression (7) et le sixième coussin sous pression (8) situé en vis-à-vis, de manière telle que lors d'un déplacement de la tête en direction d'un côté avec détection dudit mouvement par les détecteurs de pression (S9, S10) associés, le capteur de pression associé au coussin sous pression affichant la pression plus élevée abaisse la pression d'une certaine valeur et le capteur de pression associé au coussin sous pression affichant la pression moins élevée augmente la pression de la même valeur et que le cas échéant la pression dans le septième coussin sous pression (13) et le huitième coussin sous pression (14) est abaissée par les mécanismes d'entraînement (136, 146) par le déclenchement des capteurs de pression (135, 145), lorsque les détecteurs de pression (S13 ,S14) indiquent que l'utilisateur, en déplaçant sa tête vers l'arrière, exerce une pression sur lesdits septième et huitième coussins sous pression (13, 14) et est augmentée lorsque les détecteurs de pression (S13, S14) indiquent que l'utilisateur, en déplaçant sa tête vers l'avant, relâche la pression sur lesdits septième et huitième coussins sous pression (13, 14).

17. Dispositif selon une des revendications 13 à 16, **caractérisé par le fait que** l'unité de commande et de réglage (20), établit dans le premier coussin sous pression (9) ou les premières parties de coussin sous pression (91, 92), le deuxième coussin sous pression (10) ou les deuxièmes parties de coussin sous pression (101, 102), le troisième coussin sous pression (11) et le cas échéant le quatrième coussin sous pression (12), par le déclenchement des capteurs de pression (915, 925, 1015, 1025, 115, 125) et par le biais des mécanismes d'entraînement (916, 926, 1016, 1026, 116, 126), ainsi que dans le cinquième coussin sous pression (7), le sixième coussin sous pression (8), le septième coussin sous pression (13) et le cas échéant le huitième coussin sous pression (14), par le déclenchement des capteurs de pression (75, 85, 135, 145) et par le biais des mécanismes d'entraînement (76, 86, 136, 146), des forces antagonistes dans l'une ou l'autre direction d'action, qui varient en fonction de l'inclinaison de la tête sous l'action des composantes de force d'accélération G.

18. Dispositif selon une des revendications 1 à 17, **caractérisé par le fait que** les coussins sous pression ou les parties de coussin sous pression du fait des caractéristiques de leurs parois peuvent se dilater essentiellement dans la direction d'action ou dans la direction d'action supplémentaire, lorsqu'ils sont sollicités par un fluide et peuvent se contracter lorsqu'ils ne sont pas sollicités.

19. Dispositif selon la revendication 18, **caractérisé par le fait que** des éléments d'appui semi-élastiques sont prévus entre les parois s'étendant dans la direction d'action ou dans la direction supplémentaire.

20. Dispositif selon une des revendications 1 à 19, **caractérisé par le fait que** les coussins sous pression et le cas échéant les volumes d'équilibrage de pression (130) sont remplis d'un fluide, de préférence d'un liquide de type gel.

21. Dispositif selon une des revendications 1 à 20, **caractérisé par le fait que** des dispositifs de chauffage et/ou de refroidissement (S, T) sont prévus dans les coussins sous pression ou les parties de coussin sous pression, aux fins de réchauffer ou refroidir le fluide.

22. Dispositif selon une des revendications 2 à 21, **caractérisé par le fait que** la plaque de support (4) est liée à une coque de support (1) reposant sur la région des épaules de l'utilisateur.

23. Dispositif selon une des revendications 2 à 22, **caractérisé par le fait qu'**il est prévu sur la coque de support (1) un dispositif de liaison (46) pour la liaison étanche de la coque de support (1) à la partie de col supérieure d'un vêtement de protection du corps intégral.

24. Dispositif selon la revendication 23, **caractérisé par le fait que** le dispositif de liaison (46) a la forme d'un système de joint expansible annulaire.

25. Dispositif selon la revendication 24, **caractérisé par le fait que** le dispositif de liaison (46) est disposé près de l'élément annulaire (2).

26. Dispositif selon une des revendications 1 à 25, **caractérisé par le fait que** des ensembles piston/cylindre (47) sont associés aux coussins sous pression en tant qu'unités de commande et de réglage, ensembles dont les pistons (49), lorsqu'ils sont sollicités dans une direction refoulent un fluide du cylindre (43) dans le coussin sous pression.

27. Dispositif selon la revendication 26, **caractérisé par le fait que** les ensembles piston/cylindre sont disposés sur le coussin sous pression côté extérieur ou à emplacement éloigné de celui-ci.

28. Dispositif selon une des revendications 17 à 27, **caractérisé par le fait que** le dispositif est un détecteur de force de gravité G (30), une manette de commande (40), une pJateforme gyroscopique ou un pilote automatique qui génère chaque fois un signal indiquant les composantes de force de gravité G.

29. Dispositif selon une des revendications 4 à 28, **caractérisé par le fait que** les coussins sous pression du système de coussins sous pression supplémentaire, au niveau de leur surface de contact intérieure, sont conformés en fonction du profil et de la forme des os du maxillaire inférieur et de l'os occipital.

30. Application du dispositif selon une des revendications 1 à29 dans le domaine du pilotage d'avions à réaction et pour l'éjection hors de ceux-ci, dans le domaine du vol acrobatique, du sport automobile et du sport motonautique.
